# EUROPEAN PATENT SPECIFICATION

(11) **EP 4 312 884 B1**
(45) Date of publication and mention of the grant of the patent: **04.11.2026**
(21) Application number: 22716113.0
(22) Date of filing: 24.03.2022
(51) Int. Cl.: A61F 2/24

(54) **A SEALING AND ANCHORING MECHANISM FOR IRREGULAR TISSUES IN A BODY**
DICHTUNGS- UND VERANKERUNGSMECHANISMUS FÜR UNREGELMÄSSIGE GEWEBE IN EINEM KÖRPER
MÉCANISME DE SCELLEMENT ET D'ANCRAGE POUR DES TISSUS IRRÉGULIERS DANS UN CORPS

(30) Priority: 24.03.2021 US 202163165175 P
(43) Date of publication of application: 07.02.2024
(73) Proprietor: SYMBIOSIS C.M. LTD, 9363033 Jerusalem (IL)
(72) Inventor: BURG, Shira, 9363033 Jerusalem (IL); SHAFRIR, Roey, 7177619 Modiin Macabim Reut (IL); BEN YOSEF, Eran, 6962644 Tel Aviv (IL); ELISHA, Elad, 6404604 Tel Aviv (IL); GREEN, Iris, Tal Shahar 7680500 (IL)
(74) Representative: Santarelli
(86) International application number: PCT/IL2022/050324
(87) International publication number: WO 2022/201158

(56) References cited:
- WO-A1-2017/151566
- WO-A1-2020/198162
- WO-A2-2011/146745
- US-A1- 2008 200 898
- US-A1- 2012 203 264
- US-A1- 2020 352 707

## Description

### TECHNOLOGICAL FIELD

The present disclosure relates to the field of sealing and anchoring mechanisms for irregular tissues in a body and to a method for sealing and anchoring of irregular anatomical structures.

### BACKGROUND

Complex anatomical structures account for numerous functional benefits, but when a need for repair or replacement of such structures arises due to disease processes, this might cause significant difficulties in the development of matching prostheses. Good examples of such highly complex structures are the two atrioventricular heart valves, namely the mitral and the tricuspid valves, both of which have variable outlines and borders. In addition to their asymmetrical saddle-like shape, they are known to have high inter-patient variability in terms of valve structure and its three-dimensional configuration. The current approach for mitral valve replacement includes surgical replacement with universally shaped, pre-shaped, and pre-sized valve implants. Trans-catheter devices are also based on universal pre-fixed structures and sizes.

US 2020/0352707 A1 discloses systems, devices, and methods for repairing a native heart valve having an anterior leaflet and a posterior leaflet between a left atrium and a left ventricle. The valve comprises a support having a contracted configuration and an extended configuration. In the contracted configuration, the support is sized to be inserted under the posterior leaflet between a wall of the left ventricle and chordae tendineae. In the extended configuration, the support is configured to project anteriorly with respect to a posterior wall of the left ventricle by a distance sufficient to position at least a portion of the posterior leaflet toward the anterior leaflet.

WO 2011/146745 discloses a delivery catheter and a method for deploying a cardiovascular prosthetic implant using a minimally invasive procedure. The delivery catheter comprises an elongate, flexible catheter body having a proximal end and a distal end, wherein the distal end has an outer diameter of 18 French or less, a cardiovascular prosthetic implant at the distal end of the catheter body, wherein the cardiovascular prosthetic implant comprises an inflatable cuff and a tissue valve coupled to the inflatable cuff, and at least one link between the catheter body and the cardiovascular prosthetic implant.

US 2012/0203264 A1 discloses systems and methods for treating aneurysms by filling at least one double-walled filling structure, that surrounds a lumen for blood flow therethrough, with a filling medium within the aneurysm. The filling structure has a membrane that allows water molecules to permeate across the membrane in response to a water potential differential across the membrane. The transport of fluid across the membrane allows the membrane to expand or shrink to accommodate changes in the size or shape of the aneurysm, thereby maintaining a seal between the filling structure against the vessel wall and maintaining apposition of the filling structure against the inside surface of the aneurysm.

### GENERAL DESCRIPTION

To overcome the variabilities related above, there is a need in the art to provide an implant structure, conforming to various anatomies allowing optimal sealing and tissue anchoring, while minimizing tissue and adjacent anatomical structures damage. The technique of the presently claimed subject matter provides an accurate fitting for optimal sealing and anchoring to a complex anatomical site in need of a prosthesis such as the mitral valve. More specifically, the technique of the presently claimed subject matter provides a mechanism for sealing and anchoring of irregular anatomies in real time. The invention is directed to a prosthetic valve system as defined in independent claim 1.

According to a first aspect of the disclosure, there is provided a supporting structure for accommodating a prosthetic valve aimed at replacing a mitral and/or tricuspid valve and/or semilunar valves such as the aortic and pulmonic valves. The supporting structure may be configured as a mediator/spacer/adaptor/docking station between the valve's annular anatomy and a generic (i.e. round symmetrical commercially available) biosynthetic valve and/or synthetic valves and accommodates itself to the patient's valvular anatomy via shaping of a flexible material in real-time (i.e. during the replacement of the valve). This supporting structure creates a flexible and dynamic structure conforming with various and multiple valvular anatomies and may shape according to the annular anatomy.

In some embodiments, the supporting structure can accommodate prosthetic valve leaflets thus creating a fully functional valve. The supporting structure comprises a flexible carrying element defining an open cavity and being capable of accommodating a plurality of shapeable elements and a plurality of reinforcing expandable elements, such that the plurality of shapeable elements and the plurality of reinforcing expandable elements are completely enclosed within the flexible carrying element to thereby create a highly compressible, flexible dynamic supporting structure. The flexible carrying element may be made of an elastic material up to the extent enabling predominantly radial expansion upon filling of the plurality of shapeable elements. In this connection, it should be noted that the term "flexible material" refers to an elastic-plastic material being specially configured to have a deformation range having elastic physical and thermoplastic properties and a deformation range having plastic physical properties. The term "flexible structure" refers to the mechanical properties of the overall structure being configured to enable adaptation to any physical anatomy and to the capability to bend or to be bent easily without breaking. The flexibility may be variable across the different portions of the flexible carrying element and the entire flexible and dynamic structure. For example, the flexible carrying element may be divided into three portions, each portion having a different flexibility. The term "compressible structure" refers to the mechanical properties of the overall structure being configured to be compressed (i.e. collapsed) and reduced in size and/or volume. The plurality of reinforcing expandable elements provides the stability of the entire structure over time. The plurality of shapeable elements may be configured as one independent overall unit having a plurality of compartments being connected one to the others or as several separate units.

As described above, the presently disclosed subject matter relates to a minimally invasive trans-catheter replacement of a valve, such as the mitral and/or the tricuspid valve and/or semilunar valves such as the aortic and/or pulmonic valves. The supporting structure of the presently disclosed subject matter is capable of being deployed onto the native mitral valve via a trans-apical or a trans-septal approach. However, the technique of the present disclosure is not limited to the replacement of the mitral and/or the tricuspid valve and may also be applied for the treatment of any other leaking or stenosed heart valve as the semilunar valves, for closure and sealing of congenital cardiac defects such as atrial or ventricular septal defects or patent ductus arteriosus, or any other arteriovenous fistulae or shunts, and optimal left-atrial appendage sealing and closure. Moreover, the sealing and anchoring technique of the presently claimed subject matter may be applicable for optimal tissue attachment in ablation procedures of complex and tortuous anatomical structures such as the atria, pulmonary veins, renal arteries, etc. In addition, this concept is applicable for orthopedic implants, plastic surgery, neurosurgery, soft tissue surgery, etc. Additional applications for the concept of *in-situ* patient-specific prosthetic fitting and sealing may also apply.

The presently disclosed subject matter includes a collapsed symmetrical or non-symmetrical supporting structure. Once the supporting structure is deployed and its correct location is confirmed via appropriate imaging modalities such as fluoroscopy and or echocardiography, the supporting structure is filled *in-situ* according to the patient's anatomy with a biocompatible material of choice including at least one of the following suitable material in form of gas (such as helium, carbon dioxide or other), liquid (such as saline, water for injection, a liquid polymer compound with possible hardening properties such reverse thermal gels, hydrogel, medical-grade silicone or other fluids), or any other suitable material (such as a biological matrix of any kind, hyperosmotic granules, etc.) while maintaining flexibility and allowing harmonious movement with the cardiac cycle. More specifically, the flexible carrying element is capable of being shaped *in-situ.*

In some embodiments, the flexible carrying element has an external surface having a rough texture enabling to self-anchor the supporting structure to native tissue. The external surface may define any possible pattern such as a mesh-like pattern, a dot-like pattern, or a grid-like pattern. The external surface may be made of a material promoting rapid endothelial growth. The flexible carrying element may have an internal surface interfacing the open cavity being made of a material allowing smooth and linear blood flow and preventing thrombogenicity and turbulent flow. The flexible carrying element may comprise at least one non-thrombogenic fabric mesh portion.

In some embodiments, each of the plurality of the shapeable elements is configured for being adjustable *in-situ* in size and shape for self-anchoring and sealing the supporting structure to native tissue. Each of the at least one shapeable element is capable of being filled *in-situ* separately or simultaneously using at least one filling material enabling an optimal sealing and tissue anchoring to a mitral and/or tricuspid and/or semilunar valves while maintaining flexibility and allowing movement with a cardiac cycle. The filling material may have inherent flexibility property allowing eccentric widening and expansion during *in-situ* filling according to a patient's anatomy. The at least one filling material may comprise at least one fluid being in the form of at least one gas, liquid, gel, powder, granules, or any combination thereof.

In some embodiments, the plurality of reinforcing expandable element is configured and operable to self-anchor and stabilize the supporting structure. Some of the reinforcing expandable elements may be positioned on top or above a shapeable element forcing each shapeable element to expand radially. One of the reinforcing elements may define a fixed inner dimension of the open cavity and is configured and operable to maintain stability at an annular level. In this connection, it should be noted that the reinforcing elements maintain the device at fixed radius only at the waist (annular level). The fabric and the shapeable element allow mainly outer, but also an inner expansion at the atrial and ventricular level. Inner expansion may thus contribute to the sealing between the biosynthetic valve and the device at these levels which are more prevalent to leaks. Furthermore, this inner expansion does not distort the biosynthetic valve (since the expansion is with the shapeable element which is configured to conform to the structure it comes in contact with and to be not aggressive in its nature).

At least some of the plurality of reinforcing expandable elements may have the same or different physical properties including at least one of shape or diameter.

In some embodiments, each of the at least shapeable elements and of the plurality of reinforcing elements has a configuration providing at least 270 degrees sealing between the supporting structure and a native tissue according to a patient's anatomy.

In some embodiments, each of the at least shapeable elements and of the plurality of reinforcing elements has a closed-loop configuration providing substantially 360 degrees sealing between the supporting structure and a native tissue according to a patient's anatomy.

According to another broad aspect of the present invention, there is provided a prosthetic valve system comprising the supporting structure as defined above and a plurality of prosthetic valve leaflets being coupled with the supporting structure, wherein the plurality of prosthetic valve leaflets is configured and operable as a one-way valve.

In some embodiments, the plurality prosthetic valve leaflets is pre-anchored and attached to the supporting structure. Alternatively, the plurality prosthetic valve leaflets is integrated into the supporting structure.

According to another broad aspect of the present disclosure, there is provided a method for sealing between native tissue and a prosthetic implant. The method comprises: advancing and deploying a flexible collapsed supporting structure having a plurality of shapeable elements onto a native mitral valve and/or tricuspid valve and/or semilunar valves via a trans-apical or a trans-septal procedure; filling at least one shapeable element of the flexible supporting structure with a filling material in-situ; increasing an outer external surface of the flexible supporting structure; and forming the final outer shape of the flexible supporting structure according to a patient's anatomy to thereby create sealing between the native mitral valve and/or tricuspid valve and/or semilunar valves and the flexible supporting structure.

In some embodiments, filling at least one shapeable element of the flexible supporting structure with a filling material *in-situ* comprises filling *in-situ* a plurality of shapeable elements separately or simultaneously.

In some embodiments, the method further comprises self-anchoring the supporting structure to native tissue.

According to another broad aspect of the present invention, there is provided a kit for implanting a prosthetic valve, the kit comprising: a container capable of providing a sterile barrier; a sterile catheter being accommodated within the container, the sterile catheter having a distal end, and a proximal end; a sterile prosthetic valve being accommodated within the container removably coupled to the distal end of the sterile catheter, wherein the sterile prosthetic valve includes a plurality of prosthetic valve leaflets being coupled with a flexible collapsed supporting structure as defined above, and a filling material being capable of filling the flexible collapsed supporting structure and deploying the sterile prosthetic valve onto a native mitral valve and/or tricuspid valve and/or semilunar valves.

In some embodiments, the kit further includes an injection device being coupled to the flexible collapsed supporting structure. The injection device is used for injecting the material used for filling and the flexible supporting structure according to a patient's anatomy.

In some embodiments, the injection device comprises a multi-lumen delivery structure having a plurality of lumens, wherein one lumen of the multi-lumen structure is configured for injection of a filling material, the lumen comprising at least one injection port being connected to at least one shapeable element of the flexible collapsed supporting structure.

In some embodiments, at least one injection port is configured as a unidirectional port being capable of closing.

In some embodiments, the flexible collapsed supporting structure is capable of being prefilled with at least a part of the filling material.

In some embodiments, the filling material comprises a hyperosmotic agent, being capable of expanding within the flexible collapsed supporting structure upon exposition with liquid.

In some embodiments, the sterile prosthetic valve comprises a plurality of prefixed prosthetic valve leaflets being configured as a permanent valve combined, wherein the plurality prefixed prosthetic valve leaflets is accommodated within the flexible collapsed supporting structure.

In some embodiments, the plurality of prosthetic valve leaflets is pre-anchored and attached to the flexible collapsed supporting structure. Alternatively, the plurality of prosthetic valve leaflets is integrated into the flexible collapsed supporting structure.

According to another broad aspect of the present disclosure, there is provided a medium to be used with a supporting structure for accommodating a prosthetic valve, the medium comprising an external surface being made of a rough texture enabling to self-anchor the supporting structure to native tissue and an opposite internal surface being made of a material allowing smooth and linear blood flow and preventing thrombogenicity and turbulent flow across the valve.

In some embodiments, the opposite internal surface defines an open cavity.

In some embodiments, the external surface defines a mesh-like pattern, a dot-like pattern, or a grid-like pattern.

In some embodiments, the external surface is made of material promoting rapid endothelial growth.

In some embodiments, the medium is made of an elastic material, enabling predominantly radial expansion of the supporting structure upon shaping. The medium (i.e. fabric) can be configured to mostly allow expansion towards the native annulus.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** is a schematic perspective view of a possible configuration of the deployed supporting structure according to some embodiments of the presently disclosed subject matter;
**Figs. 2A-2C** are top views of different possible configurations of one shapeable element of the deployed supporting structure according to some embodiments of the presently disclosed subject matter;
**Figs. 2D-2F** are isometric views of possible configurations of the deployed supporting structure according to some embodiments of the presently disclosed subject matter;
**Figs. 3A-3D** are schematic inner front views of different possible configurations of the inner elements of the deployed supporting structure according to some embodiments of the presently disclosed subject matter;
**Figs. 4A-4D** are schematic cross-sectional views of different possible configurations of the deployed supporting structure according to some embodiments of the presently disclosed subject matter;
**Figs. 5A-5D** are side views of possible configurations of medium which may be used with a supporting structure for accommodating a prosthetic valve according to some embodiments of the presently disclosed subject-matter; **Figs. 5A1-5D1** are enlarged views of medium of **Figs. 5A-5D****;**
**Fig. 6A** is a perspective atrioventricular view of a possible configuration of the deployed supporting structure in the mitral valve according to some embodiments of the presently disclosed subject matter;
**Figs. 6B-6D** are perspective atrial views of the mitral valve **(****Fig. 6B****),** of a possible configuration of the deployed pre-shaped supporting structure in the mitral valve **(****Fig. 6C****),** of a possible configuration of the deployed shaped supporting structure in the mitral valve **(****Fig. 6D****)** according to some embodiments of the presently disclosed subject-matter;
**Figs. 6E-6F** are perspective views of a possible configuration of the deployed pre-shaped supporting structure in the tricuspid valve **(****Fig. 6E****),** of a possible configuration of the deployed shaped supporting structure in the tricuspid valve **(****Fig. 6F****)** according to some embodiments of the presently disclosed subject-matter;
**Figs. 7A-7D** are schematic different views of a possible configuration of a prosthetic valve system according to some embodiments of the presently disclosed subject-matter;
**Fig. 8** is a block diagram illustration the main steps of a method for sealing between a native tissue and a prosthetic implant according to some embodiments of the presently disclosed subject-matter; and
**Fig. 9** is a block diagram illustration a kit for implanting a prosthetic valve according to some embodiments of the presently disclosed subject-matter.

### DETAILED DESCRIPTION OF EMBODIMENTS

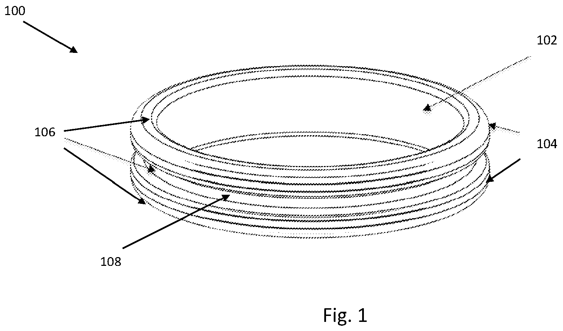

Reference is made to **Fig. 1** showing a schematic overview of a deployed anchoring and sealing supporting structure **100** for accommodating a prosthetic valve according to a broad aspect of the presently disclosed subject matter. Supporting structure **100** comprises a flexible carrying element **102** defining an open cavity and being capable of accommodating a plurality of shapeable elements **104** and a plurality of reinforcing expandable elements **106,** such that the plurality of shapeable elements **104** and the plurality of reinforcing expandable elements **106** are completely enclosed within the flexible carrying element **102** to thereby create a highly compressible, flexible dynamic supporting structure. In a specific and non-limiting example, supporting structure **100** may be configured as a collapsed symmetrical or non-symmetrical hour-glass structure. Flexible carrying element **102** is configured and operable to connect between the elements of the supporting structure and hold them together, thereby creating a highly compressible, flexible dynamic structure. The flexible dynamic structure is capable of adjusting to myocardial torsion and tissue remodeling if needed (e.g. the dynamic structure remains flexible, and thus conforms to anatomical changes after the procedure). Flexible carrying element **102** may be configured as an annular casing (i.e. ring-shaped flexible casing) and may be made of biocompatible and non-thrombogenic fabric having a mesh-like pattern. Each of the at least one shapeable element **104** is configured for being adjustable *in-situ* in size and shape for self-anchoring and sealing the supporting structure to native tissue. In particular, the plurality of shapeable elements **104** is configured for paravalvular leak prevention and real-time shaping of the supporting structure frame according to the patients' anatomy. More specifically, the plurality of shapeable elements **104** is configured as expandable sleeves being capable of being filled and shaped *in-Situ* with any biologically safe, inert, durable, and non-thrombogenic filling material of choice, so that in case of any leakage, no harm is inflicted. The filling material or part of it may be present in the collapsed supporting structure before insertion. The filling process shapes supporting structure **100** according to the patient's specific anatomy using the patient's tissue as a mold for the final shaping of the structure and acts as a personalized and accurate sealing mechanism minimizing paravalvular leaks. While filling the shapeable elements **104,** the outer diameter of each shapeable element **104** increases and forms its final outer shape *in-situ* according to the native valvular anatomy. The filling material defines inherent flexibility allowing eccentric widening and expansion during *in-situ* filling controlled by the operator. This enables accurate shaping of the supporting structure outer wall according to the patient valvular anatomy, creating a maximal seal between the native valve and the supporting structure, minimizing paravalvular leaks while minimizing damage to adjacent anatomical structures. The plurality of reinforcing expandable elements **106** is engulfed/embedded by/with the flexible carrying element **102** and is aimed at stabilizing supporting structure **100** across an irregular tubular structure (e.g. a mitral valve) acting as a scaffold. The plurality of reinforcing expandable elements **106** maintains a fixed inner dimension of supporting structure **100,** thus avoiding valvular space compromise. Shapeable elements **104** can expand anteriorly (e.g. towards the septum), posteriorly (e.g. towards the free wall) and engulf the native annulus on both its atrial and ventricular aspects whilst avoiding expansion on the expanse of atrial and/or ventricular cavity or walls (in particular avoiding left ventricular outflow tract obstruction). The plurality of reinforcing expandable elements **106** may be made of nitinol (or any other compatible material). The plurality of reinforcing expandable elements **106** generates an annular waist **108** for the stability of the flexible dynamic structure. The structural characteristics of supporting structure **100** allow for a waist **108** to be formed, anchoring and engulfing the native annulus in all its aspects.

In some embodiments, the inner diameter wall of the supporting structure is stiffer than the wall that is in direct contact with the anterolateral and posteromedial aspects of the valve, allowing for expansion only in the anterolateral-posteromedial directions, promoting waist formation and maximal seal. The supporting structure inner diameter wall may be stiffer and more durable to filling pressures, enabling a fixed inner diameter, thus avoiding concentric expansion and cavitary volume compromise on either side of the valve annulus. An additional nitinol (or any other suitable material) support may be added to the inner supporting structure wall for further reinforcement.

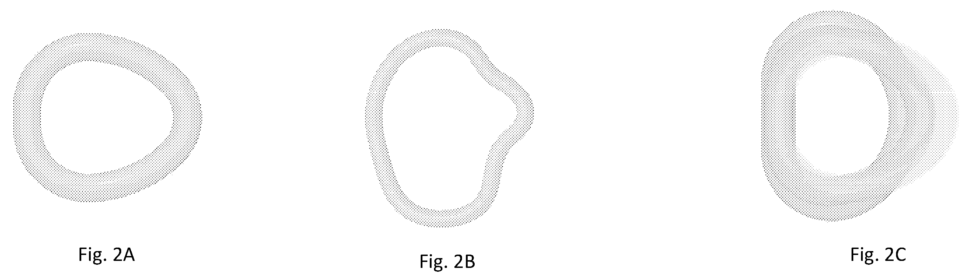

Reference is made to **Figs. 2A-2C** showing top views of different possible configurations of one shapeable element of the deployed supporting structure according to some embodiments of the presently disclosed subject matter. As described above, the shapeable element is a closed-loop element not limited to any particular geometrical configuration. The shapeable element may have various shapes, sizes, or widths. Each shapeable element of the plurality of shapeable elements may be a different one from the other or identical. The shapeable element may have asymmetrical shape.

In some cases, irregular anatomical structures are situated close to important anatomical structures (i.e. left ventricular outflow tract anteriorly to the mitral valve), thus stressing the importance of leaving these structures unharmed. For this reason, the shapeable elements of the presently disclosed subject matter may only allow radial expansion in specific vectors whilst avoiding delicate structures. As shown in the non-limiting example of **Fig. 2A****,** the shapeable element may have a non-symmetrical shape including a circular portion and an elliptical portion (D-shape). Alternatively, as shown in the non-limiting example of **Fig. 2B****,** the shape of the shapeable element may define a bulge bending outward the circular/elliptical portion. The configuration shown in **Fig. 2C****,** in which the shapeable element has a symmetrical shape including an elliptical portion and a bulge portion enables to provide 270 degrees of sealing because of the anterior portion of the shapeable material may be semi-rigid, thus not allowing expansion of the flexible material in this specific vector. This 270-degree sealing of the shapeable element facilitates an optimal sealing effect between the structure and the native tissue while avoiding contact and/or harm with/to adjacent anatomical structures.

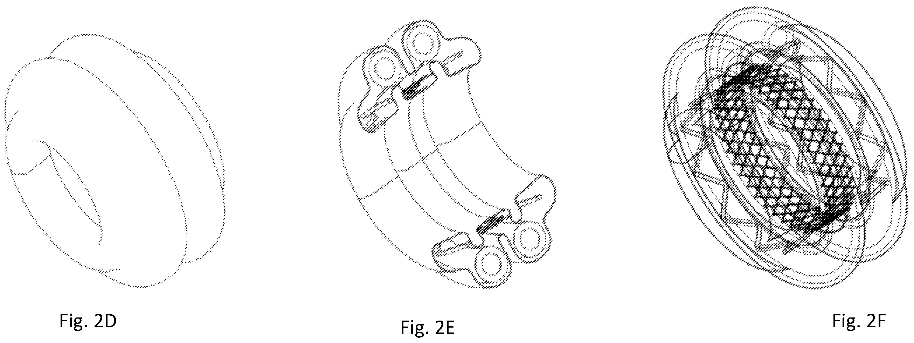

Reference is made to **Figs. 2D-2F** showing isometric views of a possible configuration of the deployed supporting structure according to some embodiments of the presently disclosed subject matter. More specifically, an external view of the whole deployed supporting structure is shown in **Fig. 2D****.** A cross-sectional view of the deployed supporting structure, showing a cross-sectional view of the inner elements: the shapeable elements and the reinforcing expandable elements surrounded by the flexible carrying element is shown in **Fig. 2E. Fig. 2F** shows an external view of the whole deployed supporting structure including the shapeable elements and the reinforcing expandable elements without the flexible carrying element.

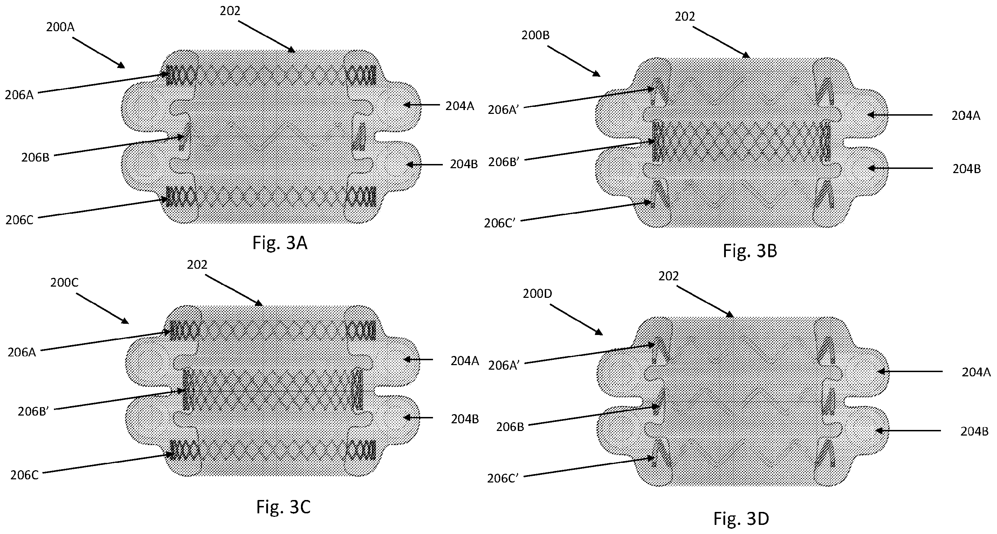

Reference is made to **Figs. 3A-3D****,** showing inner front views of a possible configuration of the different elements of the deployed supporting structures **200A-200D** according to some embodiments of the present disclosure. The plurality of reinforcing expandable elements **206A, 206A', 206B, 206B', 206C** and **206C'** as well as the shapeable elements **204A** and **204B** are engulfed/embedded by/with the flexible carrying element **202.** In a specific and non-limiting example, each of the plurality of reinforcing expandable elements **206A, 206B,** and **206C** may have a semi-open or closed-loop configuration providing annular stability. As illustrated in **Figs. 3A-3D****,** the plurality of reinforcing expandable elements **206A, 206A', 206B, 206B', 206C** and **206C'** may have different or identical shapes and/or diameters and/or widths. In this specific and non-limiting example, the plurality of reinforcing expandable elements **206A, 206B,** and **206C** may be configured as three flexible closed-loop or semi-open circular elements **206A, 206B,** and **206C** (i.e. atrial, annular, and ventricular) made of any stiff and flexible material such as nitinol. In this specific and non-limiting example, the closed-loop or semi-open circular elements may have a stent-like pattern as illustrated in **206A, 206C** or sinusoidal pattern as illustrated in **206A', 206B, 206C'** allowing flexibility and conformability. This way, it allows stability on the one hand and relative flexibility, since the plurality of reinforcing expandable elements **206A, 206A', 206B, 206B', 206C** and **206C'** is able to expand (due to its pattern-e.g. stent like or sinus) its diameter in response to the radial force applied by the opening of the biosynthetic valve.

In a specific and non-limiting example, the atrial ring **206A** or **206A'** is situated above shapeable element **204A,** allowing its compression towards the annulus during inflation, thus reducing global inflation and promoting radial expansion towards the circumferential borders of the valve at the atrial level. The ventricular ring **206C** or **206C'** is situated below shapeable element **204B,** allowing its compression towards the annulus during inflation, thus reducing global inflation and promoting radial expansion towards the circumferential borders of the valve at the ventricular level. Therefore, in this example, atrial ring **206A** or **206A'** and ventricular ring **206C** or **206C'** are configured for forcing each shapeable element **204A** and **204B** to expand radially. The annular ring/waist **206B or 206B'** is situated between the two shapeable elements **204A** and **204B** and may comprise one or more elements. The plurality of shapeable elements **204A** and **204B** may be configured as two expandable fabric sleeves, each being accommodated on distal ends of supporting structure **200A-200D** (e.g. at the atrial and ventricular level). The two expandable fabric sleeves **204A** and **204B** may contain any flexible or flexible filling material such as physiological liquids, gas, reverse thermal gels, hydrogels, hyperosmotic granules, medical-grade silicone, liquid polymers changing their physical state in response to different chemical reactions such as UV light, reaction with an additional liquid or non-liquid polymer, temperature, etc. However, the present disclosure is not limited to any use of specific filling material.

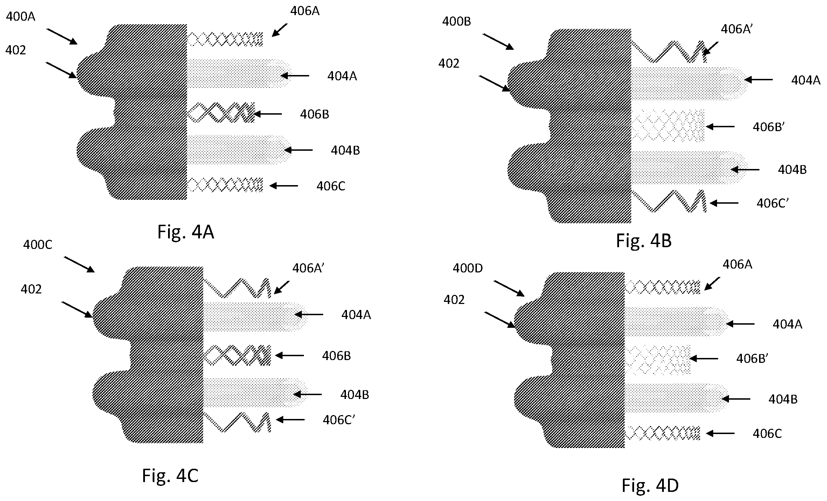

Reference is made to **Figs. 4A-4D** showing cross-sectional views of possible different configurations of the supporting structure **400A-400D** according to some embodiments of the presently disclosed subject matter. The figures show a cross-sectional view of the supporting structure **400A-400D** showing the flexible carrying element **402** covering at one side the plurality of reinforcing expandable elements **406A, 406A', 406B, 406B', 406C'** and **406C** and the plurality of shapeable elements **404A** and **404B** and at the other side the exposed plurality of reinforcing expandable elements **406A, 406A', 406B, 406B', 406C'** and **406C** and the plurality of shapeable elements **404A** and **404B.** The plurality of reinforcing expandable elements **406A, 406A', 406B, 406B', 406C'** and **406C** may be configured as a scaffold and/or may have exposed sharp edges made to be anchored within the surrounding tissue, either in the atrial, ventricular or both aspects of the valve for better tissue self-anchoring and location stability. The plurality of shapeable elements **404A** and **404B** and the flexible carrying element **402** may have the same characteristics as described above.

According to another broad aspect of the present disclosure, there is provided a medium to be used with a supporting structure for accommodating a prosthetic valve. The medium may be used with the supporting structure as described above or with any other commercially available supporting structure and/or prosthetic valve. The inventors have found that by using a medium having an external surface being made of a rough texture and an opposite internal surface being made of a material allowing smooth and linear blood flow enables to self-anchor the supporting structure to a native tissue as well as preventing thrombogenicity and turbulent flow across the valve. The flexible carrying element defines the supporting structure outer wall and may have an external surface having a rough texture for significant friction to self-anchor the supporting structure to the native tissue and allow rapid epithelialization. This trait may also be applied to generate micro-injury to the annular semi-fibrotic tissue, thereby further promoting rapid epithelialization. In particular, the external/outer surface of the medium coming in contact with the native tissue may be made of a meshwork composed of materials and constructed in a way promoting rapid endothelial growth and reduce or prevent residual leak such as a plurality of knitted interconnected loops, *inter alia* interlocked knitting or woven or any combination thereof. The knitted mesh may further comprise a tubular wall that substantially surrounds an exterior surface of the flexible structure. The knitted or woven mesh may be made by using any commercially available technique such as braiding, interlacing, electrospun, and/or dipping a porous mold into one or more reagents. The mesh may be single or multilayered, wherein each layer may have (or not) a different texture and/or structure. For example, at least a portion of the knitted/weaved/braided/ electrospun mesh is based on a single fiber which may comprise multiple filaments. At least a portion of the mesh is comprised of an elastomeric material such as polyurethane fibers which may be mixed with additional materials for strength, durability, elasticity, antibacterial properties, etc. Furthermore, in a specific and non-limiting example, the meshwork may be covered with a cell-adhesion promoting material by attachment to plasma polymers via amine and/or hydroxyl groups binding. The opposite internal surface of the medium may define an open cavity.

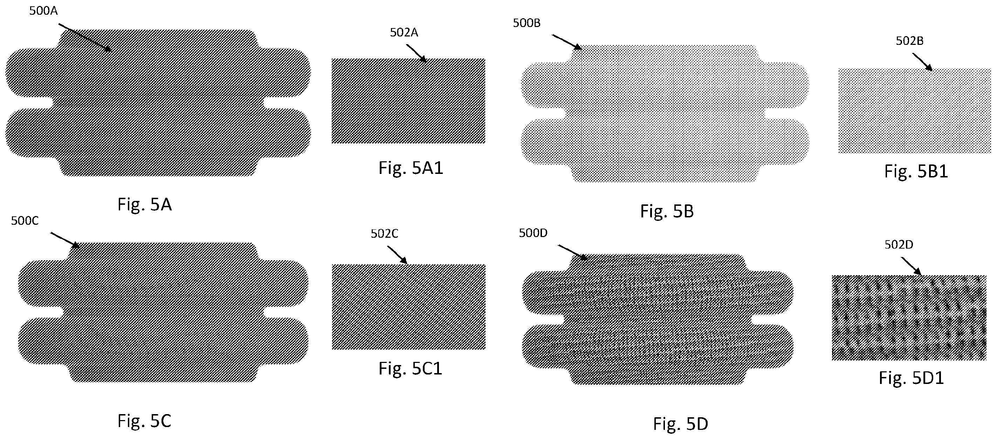

Reference is made to **Figs. 5A-5D** showing perspective views of possible configurations of the medium **500A-500D** according to some embodiments of the presently disclosed subject matter. **Figs. 5A1-5D1** are enlarged portions of the external surface of mediums **500A-500D** respectively. Medium **500A-500D** may be made of the same or different fabric portions, wherein each portion may surround a different part of the supporting structure. As shown in the figure, medium **500A-500D** may be configured as a mesh fabric lining as illustrated in **Fig. 5D1****.** However, medium **500A-500D** is not limited to such configuration. Additionally or alternatively, the external surface of medium **500A-500D** may define various fibers patterns' arrangements (e.g. along different axes) being configured to provide promoting rapid endothelial growth and reduce or prevent residual leak. For example, the fibers patterns' arrangements may include a grid-like pattern **502A** as illustrated in **Fig. 5A1****,** a rough-like pattern **502B** as illustrated in **Fig. 5B1****,** a dot-like pattern **502C** as illustrated in **Fig. 5C1****,** or a mesh-like pattern **502D** as illustrated in **Fig. 5D1****.**

If medium **502** is used with the supporting structure of the presently disclosed subject matter, medium **502** may be made of at least one material having a certain elasticity such as polyurethanes, spider silk, etc. allowing predominantly radial expansion upon modification of the shapeable element **500** within the flexible carrying element **500** (e.g. meshwork sleeve), in such manner that the filling material would not expand on the expense of the atrium or ventricle (proximally or distally to the annulus). As described above, the filling material may comprise at least one fluid being in the form of at least one gas, liquid, gel, powder, granules, or any combination thereof. The filling material may have inherent flexibility property, stability over time (at least for ten years) and should be biocompatible and non-thrombogenic such as hydrogel.

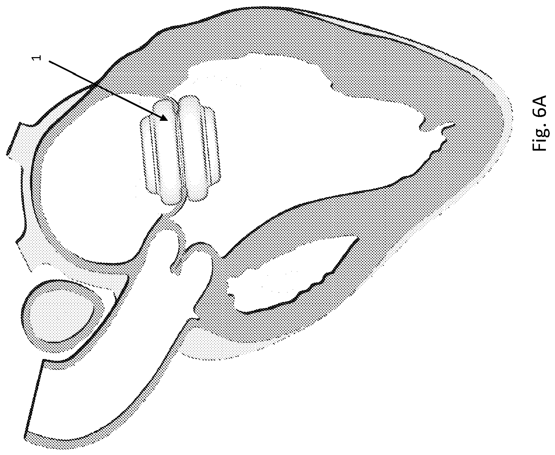

Reference is made to **Fig. 6A** showing a perspective atrioventricular view of a possible configuration of the deployed supporting structure **1** according to some embodiments of the present disclosure. The plurality of reinforcing expandable elements and the plurality of shapeable elements are not shown in this figure. The figure illustrates the functional positioning of the deployed supporting structure **1** in the mitral valve. As described above, the supporting structure of the presently disclosed subject matter may be used with any kind of separate prosthetic or bio-prosthetic valve being accommodated in the open cavity defined by the supporting structure. The supporting structure then acts as a tailor-made annular waist for ultimate sealing and self-anchoring of a prosthesis or bio-prosthesis to the native tissue.

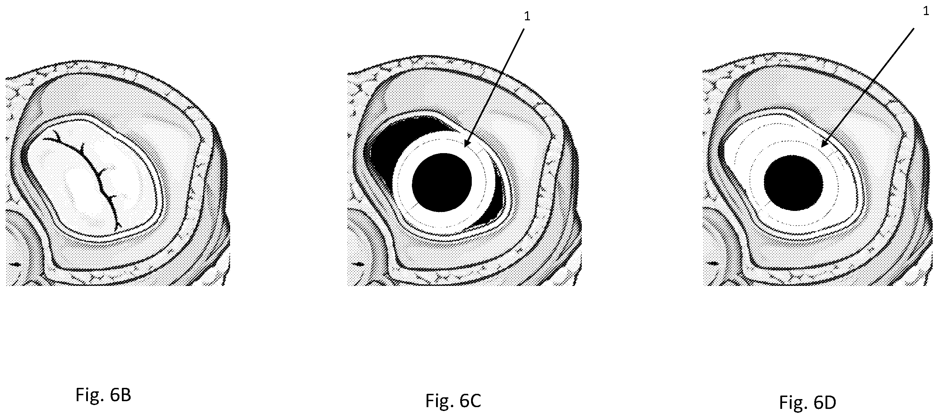

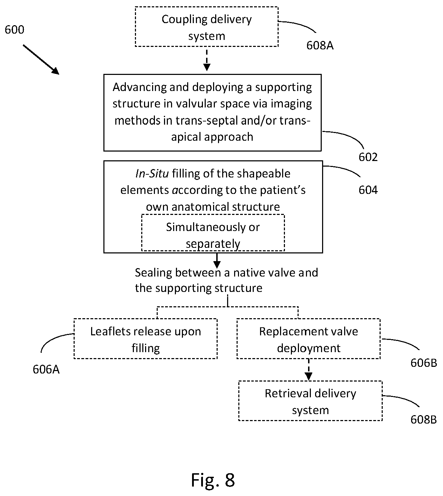

Reference is made to **Figs. 6B-6D** showing a gradual shaping process of the deployed supporting structure **1** in the mitral valve as detailed further below with respect to **Fig. 8****.** More specifically, **Fig. 6B** shows an atrial view of the mitral valve and **Fig. 6C** shows the deployed pre-shaped supporting structure **1** in the mitral valve after deployment (before shaping). **Fig. 6D** shows the shaped deployed supporting structure **1** in the mitral valve illustrating the sealing mechanism and anchoring to the irregular anatomy of the mitral valve in real-time. The capability of providing a full and optimal sealing between the deployed supporting structure **1** and the mitral valve is illustrated in the figures.

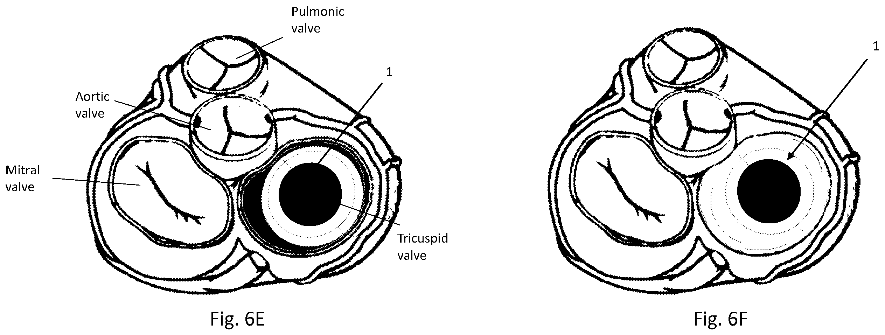

Reference is made to **Figs. 6E-6F** showing a gradual shaping process of the deployed supporting structure **1** in the tricuspid valve. More specifically, **Fig. 6E** shows the deployed pre-shaped supporting structure **1** in the tricuspid valve after deployment (before shaping). **Fig. 6F** shows the shaped deployed supporting structure **1** in the tricuspid valve illustrating the sealing mechanism and anchoring the irregular anatomy of the tricuspid valve in real time. The unique configuration of the deployed supporting structure **1** enables a full and optimal sealing and tissue anchoring for the mitral valve as well as for the tricuspid valve as illustrated in the figures, due to the capability of each of the plurality of the shapeable elements to be adjustable *in-situ* in size and in shape for self-anchoring and sealing the supporting structure to native tissue. The supporting structure 1 of the presently disclosed subject matter has the capability fit any irregular anatomies as illustrated in these two non-limiting examples.

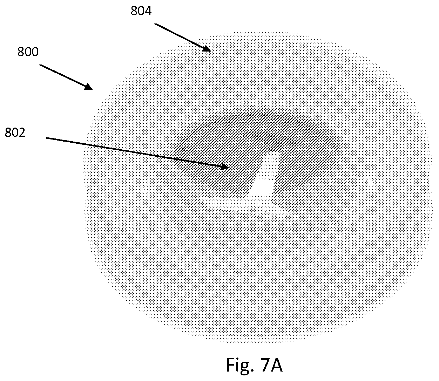

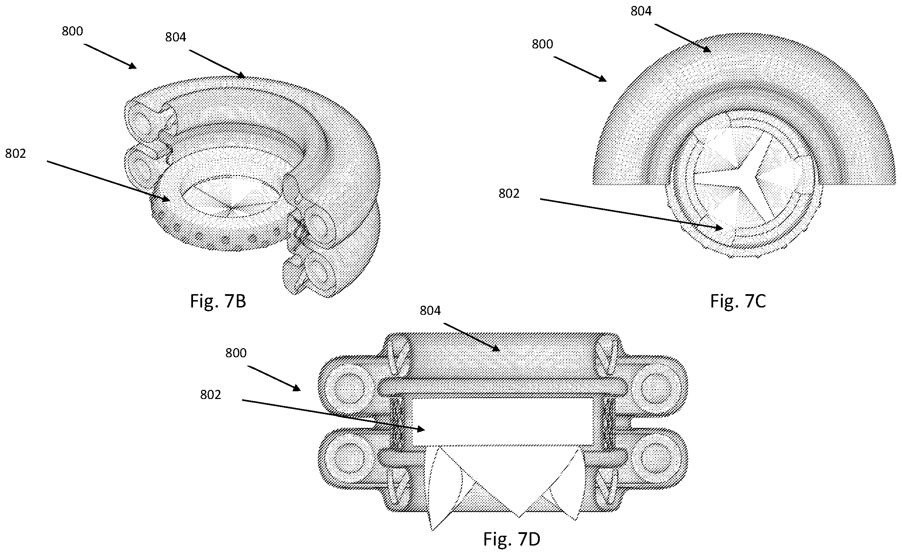

Reference is made to **Figs. 7A-7D** showing different views of a possible configuration of a prosthetic valve system **800** according to some embodiments of the present disclosure. **Fig.7A** shows a perspective view of a prosthetic valve system 800 in which valve leaflets **802** are coupled with the supporting structure **804** of the presently disclosed subject matter to provide an integrated prosthetic valve system **800.** **Figs. 7B-****7D** present respectively side, top and cross-sectional views of the same. The valve leaflets **802** may be any commercially available valve leaflets and can immediately function as a permanent valve combined with the supporting structure **804** upon its deployment and shaping. The leaflets **802** may be hidden within the supporting structure **804** before deployment. The valve leaflets **802** may be configured as embedded biological (or any other material such as printed bio-matrix, biologically inert polymers, etc.) valve leaflets. This configuration enables to provide a prosthetic valve system **800** being an independent stand-alone, fully functional prosthetic valve once deployed and shaped within the valvular space.

The valve leaflets may be configured as follows:
(i) valve leaflets may be pre-anchored/prefixed and attached to the supporting structure and may be ready to use upon deployment. Impregnated bovine/swine pericardial (or other) tissue may be used for the valve leaflets as illustrated in **Figs. 7A-7D****;**
(ii) valve leaflets may be configured as integrated biosynthetic leaflets created from biological (or other) matrix;
(iii) commercial biosynthetic valve leaflets may be integrated into the system, using the supporting structure as a sealing and anchoring waist.

According to another broad aspect, the presently disclosed subject matter relates to a kit for implanting a prosthetic valve. The kit comprises a container capable of providing a sterile barrier; a sterile catheter being accommodated within the container, the sterile catheter having a distal end and a proximal end; a sterile prosthetic valve being accommodated within the container removably coupled to the distal end of the sterile catheter, wherein the sterile prosthetic valve includes a plurality of prosthetic valve leaflets being coupled with a flexible collapsed supporting structure; and a shaping/filling material being capable of modifying and shaping the flexible collapsed supporting structure. Upon shaping of the supporting structure onto the mitral valve and/or tricuspid valve and/or semilunar valves, a sterile prosthetic valve can be delivered and deployed into the inner rim of the supporting structure. Positioning and sealing between the mitral annulus, the supporting structure and the prosthetic valve may then be confirmed by different imaging modalities. The kit may comprise an injection device being removably coupled to the flexible collapsed supporting structure. The injection device may comprise a multi-lumen delivery structure having a plurality of lumens, wherein one lumen of the multi-lumen structure is configured for injection of a filling material. The lumen comprises at least one injection port being connected to at least one shapeable element of the flexible collapsed supporting structure. The shaping material may thus be advanced through the injection port in the injection device to be reached directly into the supporting structure (containing at least one injection port) and injected in a homogenous manner. Once deployment and accurate shaping are completed with maximal seal confirmed by Trans Esophageal Echocardiography (TEE) and fluoroscopy, the injection device may be disconnected, and the ports are sealed for leaks. For example, the injection port may be configured as a unidirectional port influenced by increased hydraulic pressure within the supporting structure (or by any other closure and sealing mechanism for shaping material leakage control). Alternatively, the supporting structure may be pre-filled with a hyperosmotic agent, which expands within the supporting structure when exposed to any kind of liquid. If a liquid polymer or a gelatinous material is used as a filling material; for reducing wastage and system clogging, the injection of the filling material may be in non-penetrable closed capsules to be punctured and injected only at the proximity of the supporting structure. Alternatively, the supporting structure may be pre-filled with hyperosmotic agents which will expand within the supporting structure in exposure to any kind of liquid.

The kit dimensions can be fitted according to the specific approach (trans-apical, trans-septal, etc.). Other approaches may be applied according to the specific valve or anatomical location.

According to another broad aspect of the present disclosure, there is provided a method of *in-situ* formation of a tailor-made (i.e. patient-specific) novel sealing and anchoring mechanism for implantation of prosthetic devices in complex or irregular anatomical structures (such as a cardiac valve). This method allows precise shaping/fitting of a supporting structure outer wall according to the patient's own valvular (or other) anatomy in real-time under imaging, creating a seal between the native tissue and the supporting structure and minimizing paravalvular leaks.

Reference is made to **Fig. 8** showing a flow chart illustrating principal steps of method 600 for sealing between native tissue and a prosthetic implant. Method 600 comprises in **602** advancing and deploying a flexible collapsed supporting structure having a plurality of shapeable elements (with or without leaflets) onto a native mitral and/or tricuspid and/or semilunar valves via a trans-apical or a trans-septal approach. The collapsed supporting structure of the presently disclosed subject matter is folded into a catheter in a compressed way under vacuum. The supporting structure may comprise a plurality of shapeable elements being configured to be filled *in-situ* separately or simultaneously enabling an optimal sealing and tissue anchoring to the mitral and/or tricuspid and/or semilunar valves.

The following is a non-limiting example of a specific procedure in which the deployment of the supporting structure is gradual: the entire supporting structure is not deployed at once, but the shapeable elements are deployed in sequential steps. A femoral vein puncture is performed, and an introducer sheath is inserted into the vein. Under fluoroscopic and/or transesophageal echocardiogram (TEE) guidance, a trans-septal crossing is performed, allowing access from the groin to the left atrium of the heart. Then, a stiff wire may be inserted through the native Mitral Valve (MV) to the Left Ventricular (LV) apex. The Transseptal puncture (TSP) kit may be then retrieved, and the supporting structure delivery structure may be inserted over the wire to the LV apex. Once positioned in the LV apex, only the distal shapeable element may be exposed and then be retrieved towards the MV (while partially crimped). The distal shapeable element may then be deployed and then the supporting structure may be further retrieved towards the annulus and the annular waist is exposed as well in the MV annular level. At this stage, stability may be tested. Once confirmed, the proximal shapeable element is deployed. The at least one shapeable element is then shaped in **604** with a filling material *in-situ,* increasing the outer external surface of the flexible supporting structure; and forming its final outer shape according to the patient's anatomy to also create sealing between the native valve and the flexible supporting structure. More specifically, once the supporting structure is stable and positioned correctly under imaging modalities (TEE and/or fluoroscopy), the shaping material increases its volume by injecting a fluid media/chemical reaction. At this stage, the shapeable element reaches the optimal expansion point (controlled by the operator and may be reversible) which interacts with the native anatomy of the MV annulus and achieves an adaptive sealing.

In some embodiments, method **600** may comprise the release of a plurality of prefixed prosthetic valve leaflets being accommodated within the flexible collapsed supporting structure upon shaping in **606A.** The leaflets are configured as a permanent valve. Alternatively, method **600** may comprise a replacement valve deployment in **606B.** In this way trans-catheter implantation of a commercial prosthetic valve with a precise, patient-specific sealing mechanism is enabled. This is achieved via the final shaping of the supporting structure *in-situ* while it is already placed in its designated location, using the patient's specific anatomy as a mold. The retrieving of the supporting structure delivery structure and the insertion of the prosthetic valve may be achieved using the same guide-wire used for the delivery and deployment of the supporting structure. The prosthetic valve may be positioned while evaluating its precise location in reference to the supporting structure waist. Once the position of the prosthetic valve is confirmed under imaging modalities, the prosthetic valve is deployed and tested for stability, sealing, and functionality (i.e. paravalvular leak between the supporting structure and the prosthetic valve, high gradient, Left ventricular outflow tract obstruction (LVOTO,) leaflet coaptation).

In some embodiments, method **600** may comprise coupling in **608A** of the flexible collapsed supporting structure to a delivery system (i.e. injection device) prior to the advancement and the deployment of the flexible collapsed supporting structure in **602** and decoupling in **608B** of the flexible collapsed supporting structure to the delivery system (i.e. injection device) at the end of the procedure. The final step of the procedure includes the retrieval of the delivery system of the prosthetic valve, the retrieval of the GW and the femoral vein closure.

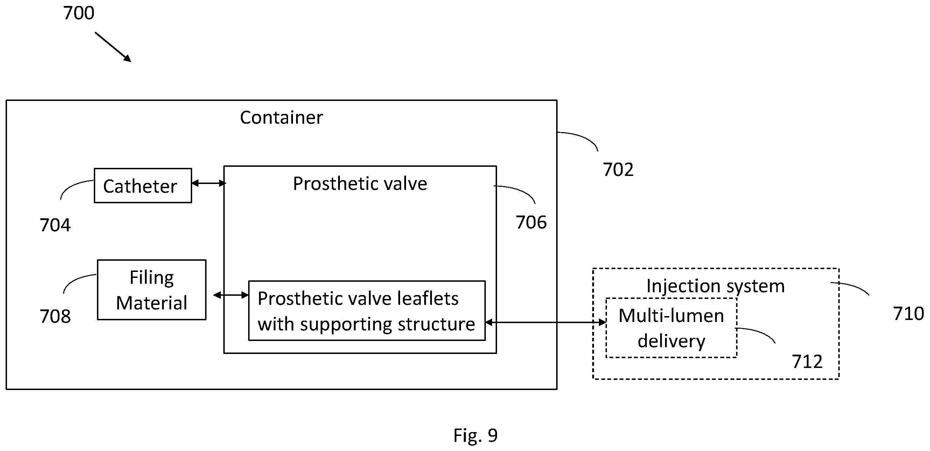

Reference is made to **Fig. 9** showing a block diagram illustrating principal functional elements of a kit **700** for implanting a prosthetic valve. Kit **700** comprises a container **702** capable of providing a sterile barrier, a sterile catheter **704** being accommodated within container **702,** a sterile prosthetic valve **706** being accommodated within container **702** removably coupled to the distal end of the sterile catheter. Sterile prosthetic valve **706** includes a plurality of prosthetic valve leaflets being coupled with a flexible collapsed supporting structure as defined above. The plurality of prosthetic valve leaflets may be accommodated within the flexible collapsed supporting structure and may be configured as prefixed prosthetic valve leaflets functioning as a permanent valve. The plurality of prosthetic valve leaflets may be pre-anchored and attached to the flexible collapsed supporting structure. Alternatively, the plurality of prosthetic valve leaflets may be integrated into the flexible collapsed supporting structure. Shaping material **708** may have inherent flexibility property, stability, and durability over time (at least for ten years) and should be biocompatible and non-thrombogenic. Shaping material **708** may comprise a hyperosmotic agent, being capable of expanding within the flexible collapsed supporting structure upon exposition with liquid. The flexible collapsed supporting structure is capable of being prefilled with at least a part of the shaping material **708.**

In some embodiments, kit **700** further comprises an injection device **710** being removably coupled to the flexible collapsed supporting structure. Injection device **710** is capable of filling and shaping the flexible supporting structure according to a patient's anatomy. Optionally, the injection device **710** may also be capable of deploying the flexible collapsed supporting structure accurately at a certain location. Injection device **710** may comprise a multi-lumen delivery structure **712** having a plurality of lumens, wherein one lumen of the multi-lumen structures is configured for injection of a filling material. The lumen comprises at least one injection port being connected to at least one shapeable element of the flexible collapsed supporting structure. The injection port may be configured as a unidirectional port being capable of closing (e.g. upon application of increased hydraulic pressure). Once the filling is completed and accurate positioning is obtained, the injection device **710** is capable of being disconnected.

## Claims

1. A prosthetic valve system comprising a supporting structure (100) for accommodating prosthetic valve leaflets, said supporting structure comprising:
a flexible carrying element (102) defining an open cavity and being capable of accommodating (i) a plurality of shapeable elements (104) each being configured for being adjustable *in-situ* in size and in shape for self-anchoring and sealing the supporting structure (100) to a native tissue, and (ii) a plurality of reinforcing expandable elements (106) being configured and operable to self-anchor and stabilize the supporting structure (100), wherein the plurality of shapeable elements (104) and the plurality of reinforcing expandable elements (106) are completely enclosed within the flexible carrying element (102) to thereby create a highly compressible, flexible dynamic supporting structure, wherein said flexible carrying element (102) is configured to be shaped *in-situ* said flexible carrying element is made of an elastic material, enabling predominantly radial expansion upon the filling of the plurality of shapeable elements,
said prosthetic valve system further comprising the plurality of prosthetic valve leaflets being coupled with the supporting structure (100), wherein the plurality of prosthetic valve leaflets is configured and operable as a one-way valve, said valve leaflets being made of 1) impregnated bovine or swine pericardial tissue, and being prefixed to the supporting structure, or 2) biologically inert polymer, and being embedded into the supporting structure.

2. The supporting structure of claim 1, wherein said flexible carrying element has an external surface having a rough texture enabling to self-anchor the supporting structure to a native tissue, and said external surface is made of a material promoting rapid endothelial growth.

3. The supporting structure of claim 2, wherein said external surface defines a mesh-like pattern or a dot-like pattern, or a grid-like pattern.

4. The supporting structure of any one of the preceding claims, wherein said flexible carrying element (102) has an internal surface interfacing the open cavity being made of a material allowing smooth and linear blood flow and preventing thrombogenicity and turbulent flow.

5. The supporting structure of any one of claims 2 to 4, wherein said flexible carrying element (102) comprises at least one non-thrombogenic fabric mesh portion.

6. The supporting structure of any one of the preceding claims, wherein each of the plurality of shapeable elements (104) is capable of being filled *in-situ* separately or simultaneously using at least one filling material enabling optimal sealing and tissue anchoring to a mitral and/or tricuspid and/or semilunar valves while maintaining flexibility and allowing movement with a cardiac cycle.

7. The supporting structure of claim 6, wherein the at least one filling material has inherent flexibility property allowing eccentric widening and expansion during in-situ filling according to a patient's anatomy, and wherein the at least one filling material comprises at least one fluid being in the form of at least one of gas, liquid, gel, powder, or granules or any combination thereof.

8. The supporting structure of one of the preceding claims, wherein one of the reinforcing expandable elements (106) defines a fixed inner dimension of the open cavity and is configured and operable to maintain stability at an annular level.

9. The supporting structure of any one of the preceding claims, wherein at least some of the plurality of reinforcing expandable elements (106) have the same or different physical properties including at least one of shape or diameter.

10. The supporting structure of any one of the preceding claims, wherein each of the at least shapeable elements (104) and of the plurality of reinforcing elements (106) has a closed-loop configuration providing substantially 360 degrees sealing between the supporting structure and a native tissue according to a patient's anatomy.

11. A kit (700) for implanting a prosthetic valve, the kit comprising:
a container (702) capable of providing a sterile barrier;
a sterile catheter (704) being accommodated within said container (702), the sterile catheter (704) having a distal end and a proximal end;
a sterile prosthetic valve (706) being accommodated within the container (702); wherein said sterile prosthetic valve (706) is removably coupled to the distal end of the sterile catheter (704), wherein the sterile prosthetic valve (706) includes a plurality of prosthetic valve leaflets being coupled to a flexible collapsed supporting structure as defined in any one of claims 1 to 11; and
a filling material being capable of filling the flexible collapsed supporting structure and deploying the sterile prosthetic valve (706) onto a native mitral valve and/or tricuspid valve and/or semilunar valves.

12. The kit of claim 11, further comprising an injection device (710) being removably coupled to said flexible collapsed supporting structure (702); said injection device (710) being capable of filling and shaping the flexible supporting structure according to a patient's anatomy.

13. The kit of claim 11 or claim 12, wherein the flexible collapsed supporting structure is capable of being prefilled with at least a part of the filling material.

## Patentansprüche

1. Prothesenklappensystem, das eine Trägerstruktur (100) zur Aufnahme von Prothesenklappenflügeln umfasst, wobei die Trägerstruktur Folgendes umfasst:
ein flexibles Tragelement (102), das einen offenen Hohlraum definiert und in der Lage ist, (i) eine Vielzahl von formbaren Elementen (104), von denen jedes so konfiguriert ist, dass es *in-situ* in Größe und Form verstellbar ist, um die Trägerstruktur (100) selbstständig in einem nativen Gewebe zu verankern und abzudichten, sowie (ii) eine Vielzahl von verstärkenden, aufblasbaren Elementen (106) aufzunehmen, die so konfiguriert und funktionsfähig sind, dass sie die Trägerstruktur (100) selbstständig verankern und stabilisieren, wobei die Vielzahl von formbaren Elementen (104) und die Vielzahl von verstärkenden, aufblasbaren Elementen (106) vollständig innerhalb des flexiblen Tragelements (102) eingeschlossen sind, um so eine hoch komprimierbare, flexible dynamische Trägerstruktur zu schaffen, wobei das flexible Tragelement (102) so konfiguriert ist, dass es *in-situ* geformt werden kann, wobei das flexible Tragelement aus einem elastischen Material besteht, das überwiegend eine radiale Ausdehnung bei dem Befüllen der Vielzahl von formbaren Elementen ermöglicht, wobei das Prothesenklappensystem ferner die Vielzahl von Prothesenklappenflügeln umfasst, die mit der Trägerstruktur (100) gekoppelt sind, wobei die Vielzahl von Prothesenklappenflügeln als Einwegventil konfiguriert und funktionsfähig ist, wobei die Klappenflügel aus 1) imprägniertem Rinder- oder Schweine-Perikardgewebe bestehen und an der Trägerstruktur befestigt sind oder 2) aus biologisch inertem Polymer bestehen und in die Trägerstruktur eingebettet sind.

2. Trägerstruktur nach Anspruch 1, wobei das flexible Tragelement eine äußere Oberfläche mit einer rauen Textur aufweist, die es ermöglicht, die Trägerstruktur selbstständig in einem nativen Gewebe zu verankern, und wobei die äußere Oberfläche aus einem Material besteht, das ein schnelles Endothelwachstum fördert.

3. Trägerstruktur nach Anspruch 2, wobei die äußere Oberfläche ein netzartiges Muster oder ein punktartiges Muster oder ein gitterartiges Muster definiert.

4. Trägerstruktur nach einem der vorhergehenden Ansprüche, wobei das flexible Tragelement (102) eine innere Oberfläche aufweist, die mit dem offenen Hohlraum in Kontakt steht und aus einem Material besteht, das einen glatten und linearen Blutfluss ermöglicht und Thrombogenität sowie einen turbulenten Fluss verhindert.

5. Trägerstruktur nach einem der Ansprüche 2 bis 4, wobei das flexible Tragelement (102) mindestens einen nicht-thrombogenen Gewebenetz-Anteil umfasst.

6. Trägerstruktur nach einem der vorhergehenden Ansprüche, wobei jedes der Vielzahl von formbaren Elementen (104) in der Lage ist, *in-situ* separat oder gleichzeitig mit mindestens einem Füllmaterial gefüllt zu werden, das eine optimale Abdichtung und Gewebeverankerung an Mitral- und/oder Trikuspidal- und/oder Taschenklappen ermöglicht, während die Flexibilität erhalten bleibt und eine Bewegung mit dem Herzzyklus ermöglicht wird.

7. Trägerstruktur nach Anspruch 6, wobei das mindestens eine Füllmaterial eine inhärente Flexibilitätseigenschaft aufweist, die eine exzentrische Erweiterung und Ausdehnung während der In-situ-Befüllung entsprechend der Anatomie des Patienten ermöglicht, und wobei das mindestens eine Füllmaterial mindestens ein Fluid umfasst, das in Form von mindestens einem von Gas, Flüssigkeit, Gel, Pulver oder Granulat oder einer beliebigen Kombination davon vorliegt.

8. Trägerstruktur nach einem der vorhergehenden Ansprüche, wobei eines der verstärkenden, aufblasbaren Elemente (106) eine feste innere Abmessung des offenen Hohlraums definiert und so konfiguriert und funktionsfähig ist, dass es die Stabilität auf einer ringförmigen Ebene aufrechterhält.

9. Trägerstruktur nach einem der vorhergehenden Ansprüche, wobei mindestens einige der Vielzahl von verstärkenden, aufblasbaren Elementen (106) dieselben oder unterschiedliche physikalische Eigenschaften aufweisen, einschließlich mindestens einer Form oder eines Durchmessers.

10. Trägerstruktur nach einem der vorhergehenden Ansprüche, wobei jedes der mindestens formbaren Elemente (104) und der Vielzahl von verstärkenden Elementen (106) eine geschlossene Schleifenkonfiguration aufweist, die entsprechend der Anatomie des Patienten eine im Wesentlichen 360-Grad-Abdichtung zwischen der Trägerstruktur und dem nativen Gewebe gewährleistet.

11. Kit (700) zur Implantation einer Prothesenklappe, wobei das Kit Folgendes umfasst:
einen Behälter (702), der in der Lage ist, eine sterile Barriere bereitzustellen;
einen sterilen Katheter (704), der in dem Behälter (702) aufgenommen ist, wobei der sterile Katheter (704) ein distales Ende und ein proximales Ende aufweist;
eine sterile Prothesenklappe (706), die in dem Behälter (702) untergebracht ist; wobei die sterile Prothesenklappe (706) abnehmbar mit dem distalen Ende des sterilen Katheters (704) gekoppelt ist, wobei die sterile Prothesenklappe (706) eine Vielzahl von Prothesenklappenflügeln umfasst, die mit einer flexiblen, zusammengefalteten Trägerstruktur gekoppelt sind, wie in einem der Ansprüche 1 bis 11 definiert; und
ein Füllmaterial, das in der Lage ist, die flexible, zusammengefaltete Trägerstruktur zu füllen und die sterile Prothesenklappe (706) auf eine native Mitralklappe und/oder Trikuspidalklappe und/oder Taschenklappen zu entfalten.

12. Kit nach Anspruch 11, ferner umfassend eine Injektionsvorrichtung (710), die abnehmbar mit der flexiblen, zusammengefalteten Trägerstruktur (702) gekoppelt ist; wobei die Injektionsvorrichtung (710) in der Lage ist, die flexible Trägerstruktur entsprechend der Anatomie eines Patienten zu füllen und zu formen.

13. Kit nach Anspruch 11 oder Anspruch 12, wobei die flexible, zusammengefaltete Trägerstruktur in der Lage ist, mindestens teilweise mit dem Füllmaterial vorbefüllt zu werden.

## Revendications

1. Système de valvule prothétique comprenant une structure porteuse (100) destinée à accueillir des feuillets de valvule prothétique, ladite structure porteuse comprenant :
un élément porteur flexible (102) définissant une cavité ouverte et étant capable d'accueillir (i) une pluralité d'éléments façonnables (104) chacun étant configuré pour être ajustable *in-situ* en taille et en forme pour l'auto-ancrage et l'étanchéité de la structure porteuse (100) à un tissu natif, et (ii) une pluralité d'éléments expansibles de renforcement (106) étant configurés et utilisables pour s'auto-ancrer et stabiliser la structure porteuse (100), dans lequel la pluralité d'éléments façonnables (104) et la pluralité d'éléments expansibles de renforcement (106) sont complètement enfermés à l'intérieur de l'élément porteur flexible (102) afin de créer une structure porteuse dynamique, flexible et hautement compressible, dans lequel ledit élément porteur flexible (102) est configuré pour être façonné *in-situ*, ledit élément porteur flexible est fabriqué en matériau élastique, permettant une expansion principalement radiale lors du remplissage de la pluralité d'éléments façonnables, ledit système de valvule prothétique comprenant en outre la pluralité de feuillets de valvule prothétique couplés à la structure porteuse (100), dans lequel la pluralité de feuillets de valvule prothétique sont configurés et utilisables en tant que valvule unidirectionnelle, lesdits feuillets de valvule étant fabriqués en 1) tissu péricardique bovin ou porcin imprégné, et étant préfixés à la structure porteuse, ou 2) polymère biologiquement inerte, et étant incorporés dans la structure porteuse.

2. Structure porteuse selon la revendication 1, dans laquelle ledit élément porteur flexible présente une surface externe présentant une texture rugueuse permettant l'auto-ancrage de la structure porteuse au tissu natif, et ladite surface externe est constituée d'un matériau favorisant une croissance endothéliale rapide.

3. Structure porteuse selon la revendication 2, dans laquelle ladite surface externe définit un motif en forme de maille, un motif en forme de points ou un motif en forme de grille.

4. Structure porteuse selon l'une quelconque des revendications précédentes, dans laquelle ledit élément porteur flexible (102) présente une surface interne en interface avec la cavité ouverte, étant constituée d'un matériau permettant un écoulement sanguin lisse et linéaire et empêchant la thrombogénicité ainsi qu'un écoulement turbulent.

5. Structure porteuse selon l'une quelconque des revendications 2 à 4, dans laquelle ledit élément porteur flexible (102) comprend au moins une partie en maille de tissu non thrombogène.

6. Structure porteuse selon l'une quelconque des revendications précédentes, dans laquelle chacun de la pluralité d'éléments façonnables (104) est capable d'être rempli *in-situ* séparément ou simultanément à l'aide d'au moins un matériau de remplissage permettant une étanchéité optimale et un ancrage tissulaire optimal aux valvules mitrales et/ou tricuspides et/ou semi-lunaires tout en maintenant la flexibilité et en permettant le mouvement avec un cycle cardiaque.

7. Structure porteuse selon la revendication 6, dans laquelle l'au moins un matériau de remplissage présente une propriété de flexibilité inhérente permettant un élargissement et une expansion excentriques lors du remplissage in situ selon l'anatomie d'un patient, et dans laquelle l'au moins un matériau de remplissage comprend au moins un fluide sous la forme d'au moins un parmi un gaz, un liquide, un gel, une poudre ou des granules ou toute combinaison de ceux-ci.

8. Structure porteuse selon l'une des revendications précédentes, dans laquelle l'un des éléments expansibles de renforcement (106) définit une dimension intérieure fixe de la cavité ouverte et est configuré et utilisable pour maintenir la stabilité à un niveau annulaire.

9. Structure porteuse selon l'une quelconque des revendications précédentes, dans laquelle au moins certains de la pluralité d'éléments expansibles de renforcement (106) présentent des propriétés physiques identiques ou différentes, y compris au moins l'un parmi une forme ou un diamètre.

10. Structure porteuse selon l'une quelconque des revendications précédentes, dans laquelle chacune parmi la pluralité d'éléments façonnables (104) et la pluralité d'éléments de renforcement (106) présente une configuration en boucle fermée assurant une étanchéité sensiblement à 360 degrés entre la structure porteuse et un tissu natif selon l'anatomie d'un patient.

11. Kit (700) pour implanter une valvule prothétique, le kit comprenant :
un récipient (702) capable de fournir une barrière stérile ;
un cathéter stérile (704) étant accueilli dans ledit récipient (702), le cathéter stérile (704) présentant une extrémité distale et une extrémité proximale ;
une valvule prothétique stérile (706) étant accueillie à l'intérieur du récipient (702) ; dans laquelle ladite valvule prothétique stérile (706) est couplée de manière amovible à l'extrémité distale du cathéter stérile (704), dans lequel la valvule prothétique stérile (706) comporte une pluralité de feuillets de valvule prothétique couplés à une structure porteuse flexible repliée selon l'une quelconque des revendications 1 à 11 ; et
un matériau de remplissage capable de remplir la structure porteuse flexible repliée et de déployer la valvule prothétique stérile (706) sur une valvule mitrale native et/ou une valvule tricuspide native et/ou des valvules semi-lunaires natives.

12. Kit selon la revendication 11, comprenant en outre un dispositif d'injection (710) étant couplé de manière amovible à ladite structure porteuse flexible repliée (702) ; ledit dispositif d'injection (710) étant capable de remplir et de façonner la structure porteuse flexible selon l'anatomie d'un patient.

13. Kit selon la revendication 11 ou la revendication 12, dans lequel la structure porteuse flexible repliée est capable d'être préremplie avec au moins une partie du matériau de remplissage.
